(19) Europäisches Patentamt
European Patent Office
Office européen des brevets

(11) **EP 1 727 580 B1**

(12) **EUROPÄISCHE PATENTSCHRIFT**

(45) Veröffentlichungstag und Bekanntmachung des Hinweises auf die Patenterteilung:
**23.01.2008 Patentblatt 2008/04**

(21) Anmeldenummer: **05733699.2**

(22) Anmeldetag: **23.03.2005**

(51) Int Cl.:
***A61M 16/00*** *(2006.01)*

(86) Internationale Anmeldenummer:
**PCT/DE2005/000534**

(87) Internationale Veröffentlichungsnummer:
**WO 2005/092414 (06.10.2005 Gazette 2005/40)**

(54) **VERFAHREN ZUR STEUERUNG EINES BILEVEL-GERÄTS SOWIE BILEVEL-GERÄT**

METHOD FOR CONTROL OF A BI-LEVEL DEVICE AND BI-LEVEL DEVICE

PROCEDE DE COMMANDE D'UN APPAREIL BI-LEVEL ET APPAREIL BI-LEVEL

(84) Benannte Vertragsstaaten:
**CH DE FR GB LI NL**

(30) Priorität: **23.03.2004 DE 102004014538**

(43) Veröffentlichungstag der Anmeldung:
**06.12.2006 Patentblatt 2006/49**

(73) Patentinhaber: **Seleon GmbH**
**06847 Dessau (DE)**

(72) Erfinder:
• **GABRIEL, Stephan**
**06849 Dessau (DE)**

• **WHITEHEAD, Ellis**
**79108 Freiburg (DE)**
• **GENGER, Harald**
**06846 Dessau (DE)**

(74) Vertreter: **Hellmich, Wolfgang**
**Ernsbergerstrasse 14**
**81241 München (DE)**

(56) Entgegenhaltungen:
**EP-A- 0 656 216          EP-A- 1 005 829**
**WO-A-98/35715          US-A- 5 433 193**
**US-A1- 2003 221 689**

**Beschreibung**

Die Erfindung bezieht sich auf ein Verfahren der im Oberbegriff des Anspruchs 1 genannten Art sowie ein BiLevel-Gerät, das ein solches Verfahren durchführt. Konkret bezieht sich diese Erfindung darauf, wie trotz der vom BiLevel-Gerät erzeugten Druckschwankungen Inspiration und Exspiration sicher erkannt werden.

BiLevel-Geräte wie auch die etwas einfacheren CPAP-Geräte dienen der pneumatischen Schienung der Atemwege um obstruktive Atemstörungen während des Schlafens zu verhindern.

Zur Behandlung von Apnoen wurde die CPAP (continuous positive airway pressure)-Therapie entwickelt, die in Chest. Volume No. 110, Seiten 1077-1088, Oktober 1996 und Sleep, Volume No. 19, Seiten 184-188 beschrieben wird. Ein CPAP-Gerät erzeugt mittels eines Kompressors oder einer Turbine einen positiven Überdruck bis zu etwa 30 mbar und appliziert diesen vorzugsweise über einen Luftbefeuchter, über einen Schlauch und eine Nasenmaske in den Atemwegen des Patienten. Dieser Überdruck soll gewährleisten, dass die oberen Atemwege während der gesamten Nacht vollständig geöffnet bleiben und somit keine Apnoen auftreten (DE 198 49 571 A1), die den Schlaf des Patienten stören. Der erforderliche Überdruck hängt unter anderem von dem Schlafstadium und der Körperposition des Schlafenden ab.

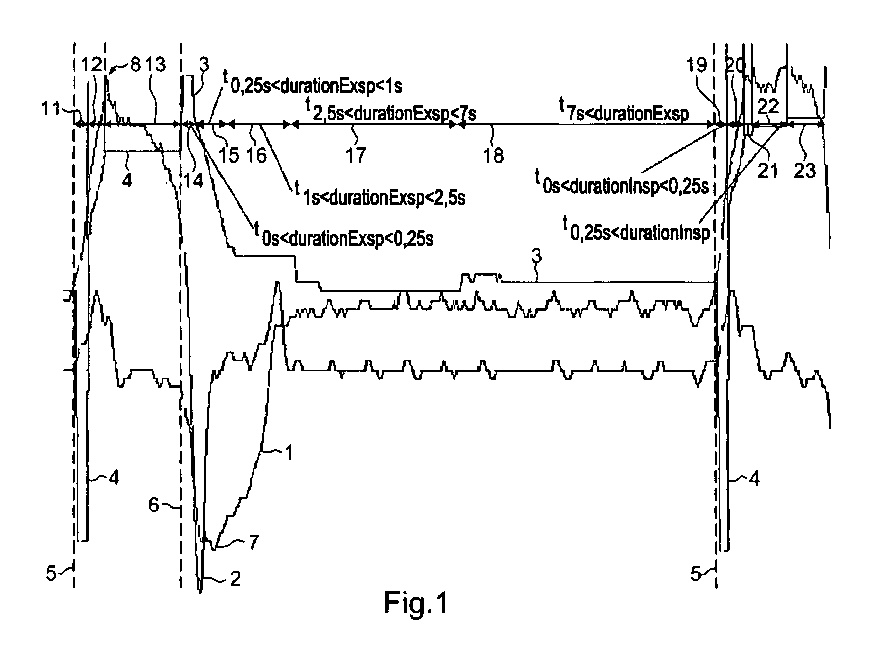

Der Überdruck wird von Patienten oft als störend empfunden. Um den Überdruck so gering wie möglich aber so hoch wie nötig einzustellen wurden so genannte Auto CPAP-Geräte (vgl. Fig. 1) entwickelt. Algorithmen zur Einstellung des optimalen Überdrucks sind beispielsweise aus den WO 00/24446 A1, WO 02/00283 A1 und WO 02/083221 A1 bekannt. Zusätzlich zum Luftdruck wie bei einfacheren CPAP-Geräten wird bei Auto CPAP-Geräten zusätzlich der Luftfluss zum Patienten gemessen. Bei der Verarbeitung des Luftflusses werden Maxima und Minima in der zeitlichen Ableitung des Luftflusses gesucht und anhand dieser Extrema Inspirations- und Exspirationsphasen bestimmt.

Ein anderer Ansatz, um die pneumatische Schienung für den Patienten möglichst wenig unangenehm zu machen, sind so genannte BiLevel-Geräte. BiLevel-Geräte unterstützen die Atmung des Patienten dadurch, dass dem Patienten während der Inspiration ein etwas höherer Druck als während der Exspiration appliziert wird.

Aufgrund der unterschiedlichen Drücke während der Inspiration und Exspiration ist es bei BiLevel-Geräten notwendig, Inspirations- und Exspirationsphasen zu bestimmen. Bei den aus den WO 98/35715 A1 und EP 0 656 216 A2 bekannten BiLevel-Geräten wird die zeitliche Ableitung des Luftflusses mit Schwellenwerten verglichen um zwischen Inspirations- und Exspirationsphasen zu unterscheiden. Laut WO 98/35715 A1 war dieses Verfahren im Stand der Technik üblich (übergreifender Absatz von Seiten 1 und 2).

Es ist bei BiLevel-Geräten technisch schwierig die Übergänge zwischen Inspiration und Exspiration exakt aufgrund des Luftflusses zu erfassen, weil genau bei diesen Übergängen der Druck geändert werden soll. Durch die Druckänderung wird die Luft im Beatmungschlauch und den Lungen des Patienten leicht komprimiert oder expandiert, wodurch dem durch die Atmung erzeugten Luftfluss ein durch die Druckänderung erzeugter Luftfluss überlagert wird. Der durch die Druckänderung erzeugte Luftfluss ist nun genau bei den Zeitpunkten besonders groß, die exakt erfasst werden sollen.

Problematisch ist auch, dass eine Ableitung wie eine Hochpassfilterung wirkt und so zu einer Aufrauhung des Signals führt. Hierbei tritt Rauschen stärker hervor. Aufgrund der Rauheit des Signals kann der einfache Vergleich mit Schwellenwerten zu falschen Ergebnissen führen. Deshalb wird in der WO 02/083221 A2 die Ableitung mit einer Tiefpassfilterung kombiniert und dies als "Schätzen der Ableitung" bezeichnet. Eine Tiefpassfilterung hat andererseits den Nachteil, dass sie den Anstieg oder Abfall eines Signals verzögert.

Als nächstliegender Stand der Technik wird das Dokument US 5 433 193 angesehen, das alle Merkmale des Oberbegriffs des Anspruchs 1 offenbart.

Es ist Aufgabe der Erfindung ein verbessertes Verfahren sowie ein verbessertes BiLevel-Gerät anzugeben, die die Zeitpunkte der Übergänge zwischen Inspiration und Exspiration genauer erfassen und damit die Inspirations- und Exspirationsphasen exakter bestimmen.

Diese Aufgabe wird durch die Lehre der unabhängigen Ansprüche gelöst.

Bevorzugte Ausführungsformen der Erfindung sind Gegenstand der Unteransprüche.

Vorteilhaft an Vergleichen des Luftflusses mit Schwellenwerten ist, dass hierbei eine Aufrauhung des Luftflusses durch eine zeitliche Ableitung sowie eine Verzögerung durch Tiefpassfilterung vermieden wird.

Das Wählen des minimalen Luftflusses während der vorangehenden Exspirationsphase als Schwellenwert unmittelbar nach dem Umschalten in einen Inspirationsmodus sowie das Wählen des maximalen Luftflusses während der vorangehenden Inspirationsphase als Schwellenwert unmittelbar nach dem Umschalten in einen Exspirationsmodus verhindert, dass aufgrund der mit der Druckänderung einhergehenden Schwankungen des Luftflusses zu schnell ein weiteres Umschalten in den anderen Atmungsmodus erfolgt.

Auch das zusätzliche Vergleichen der Ableitung des Luftflusses mit unterschiedlichen Schwellenwerten während der Inspirations- und Exspirationsphasen verhindert ein unerwünschtes Umschalten in den anderen Atmungsmodus.

Zur Rauschunterdrückung wird der gemessene Luftfluss zunächst einer Medianfilterung unterzogen und anschließend gemittelt, bevor die Ableitung des Luftflusses berechnet wird. Die Medianfilterung unterdrückt in vorteilhafter Weise Ausreißer. Die Kombination einer Medianfilterung über wenige Messwerte und eine anschließende Mittelung über die doppelte Anzahl von Messwerten stellt einen optimalen Kompromiss zwischen Rechenaufwand, Filterlaufzeit und erforderlicher Mittelung dar.

Die zusätzliche Berücksichtigung des Istdrucks erhöht die Zuverlässigkeit der Umschaltung zwischen Inspirations- und Exspirationsmodus. Ein Aspekt hierbei ist, dass bei Husten oder Niesen nicht in den Inspirationsmodus umgeschaltet werden soll. Hierbei steigt der Istdruck über den Solldruck an, weil die Druckregelschleife zu träge ist, um solche schnellen Druckschwankungen auszugleichen. Der andere Aspekt ist, dass in den Inspirationsmodus umgeschaltet werden soll, wenn der Istdruck unter dem Solldruck liegt und der Luftfluss in einer dritten vorgegebenen Zeitspanne stark angestiegen ist.

Das Absenken des Schwellenwerts für den Luftfluss während der Exspiration kurz nach dem Umschalten in den Exspirationsmodus macht das Verfahren zunehmend empfindlicher für ein Umschalten zurück in den Inspirationsmodus. Dies entspricht in vorteilhafter Weise der durchschnittlichen Dauer einer Exspirationsphase.

In entsprechend vorteilhafter Weise wird der Schwellenwert für den Luftfluss während der Inspiration näherungsweise proportional zum aktuellen Luftfluss angehoben, bis der aktuelle Luftfluss ein Maximum erreicht. Nach dem Erreichen des Maximums wird der Schwellenwert in etwa konstant gehalten.

Im Folgenden wird eine bevorzugte Ausführungsform der Erfindung unter Bezugnahme auf die beiliegenden Zeichnungen näher erläutert. Dabei zeigen:

Fig. 1 ein Diagramm mit dem zeitlichen Verlauf des Luftflusses, der Ableitung des Luftflusses sowie des Schwellenwerts für den Luftfluss;

Fig. 2 die Hardware eines AutoCPAP- oder BiLevel-Geräts;

Fig. 3 ein Flussdiagramm für das erfindungsgemäße Steuerungsverfahren für ein BiLevel-Gerät;

Fig. 4 ein detaillierteres Flussdiagramm für die Vorverarbeitung;

Figuren 5 bis 7 ein detaillierteres Flussdiagramm für die Exspirationsverarbeitung; und

Fig. 8 ein detaillierteres Flussdiagramm für die Inspirationsverarbeitung.

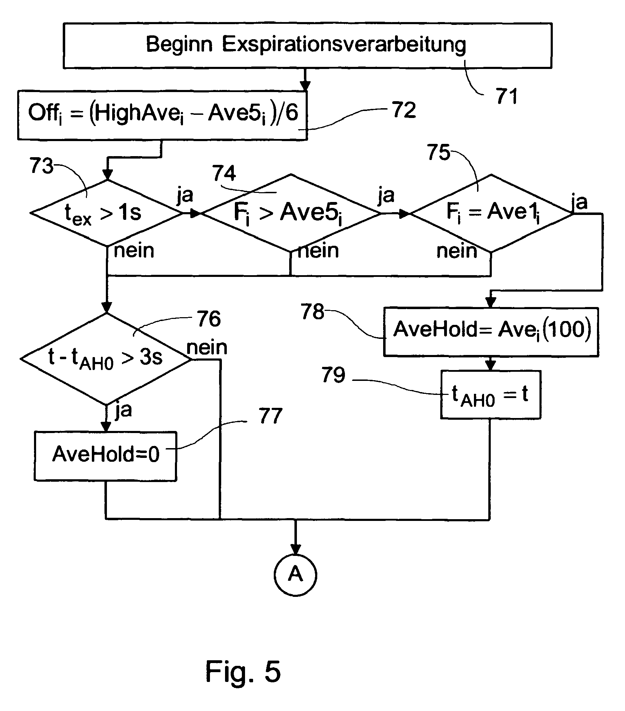

Fig. 1 zeigt ein Diagramm mit dem zeitlichen Verlauf des Luftflusses 1, der Ableitung des Luftflusses 2 sowie den Exspirationsschwellenwert 3 und den Inspirationsschwellenwert 4. Außerdem wurden unterschiedliche Zeitbereiche markiert auf die weiter unten im Zusammenhang mit dem Flussdiagramm in den Figuren 5 bis 8 genauer eingegangen wird. Die Exspirationsphase wurde von der Testperson absichtlich in die Länge gezogen, um alle, im erfindungsgemäßen Verfahren vorgesehenen Zeitbereiche darzustellen.

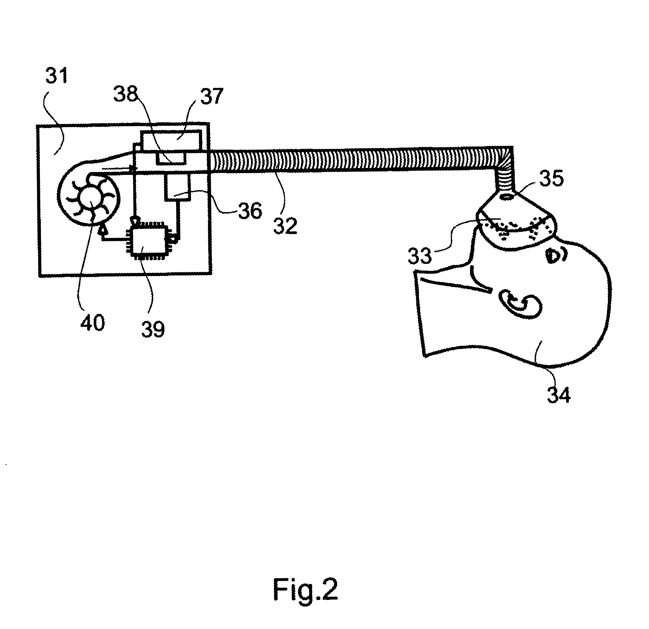

Fig. 2 zeigt schematisch die Hardware für ein AutoCPAP- oder ein BiLevel-Gerät 31. Ein Lüfter 40 fördert Luft und stellt diese unter einem Überdruck von bis zu 30 mbar zur Verfügung. Die Luft wird über einen Beatmungschlauch 32 und eine Maske 33 einem Patienten 34 appliziert. Durch die Öffnung 35 entweicht ständig Luft in die Umgebung, sodass ausgeatmete Luft mit einem hohen $CO_2$ Anteil durch die Öffnung 35 ausgespült wird. In Gerät 31 ist ein Drucksensor 36 zur Messung des vom Lüfter 40 erzeugten Überdrucks eingebaut. Dieser Überdruck wird im Folgenden als Istdruck bezeichnet. Darüber hinaus enthält Gerät 31 einen Flusssensor 37 zur Erfassung des Luftflusses. Beispielhaft ist ein Heizdraht 38 des Flusssensor 37 dargestellt. Die vom Flusssensor 37 und Drucksensor 36 gelieferten Signale werden einem Mikroprozessor 39 zugeführt. Der Mikroprozessor steuert wiederum die Drehzahl von Lüfter 40.

Der Aufbau von Gerät 31 folgt dem Trend in der Elektronik, Sensorsignale möglichst schnell zu digitalisieren und die Signalverarbeitung dann digital durchzuführen. Das von Mikroprozessor 39 abgearbeitete Programm enthält eine innere Regelschleife gemäß der die Drehzahl des Lüfters so gesteuert wird, dass der von Drucksensor 36 gemessene Istdruck möglichst gut mit einem Solldruck übereinstimmt. Der Solldruck wird von anderen Programmteilen vorgegeben. Wird der Solldruck unabhängig von Inspiration und Exspiration vorgegeben, so handelt es sich bei Gerät 31 um ein CPAP-Gerät. Wird zusätzlich der Luftfluss ausgewertet, um den Solldruck zu optimieren, handelt es sich um ein AutoCPAP-Gerät. Werden unterschiedliche Solldrücke für Inspiration und Exspiration vorgegeben, so handelt es sich bei Gerät 31 um ein BiLevel-Gerät. Es hängt also lediglich von den anderen Programmteilen ab, ob Gerät 31 als AutoCPAP- oder als BiLevel-Gerät arbeitet.

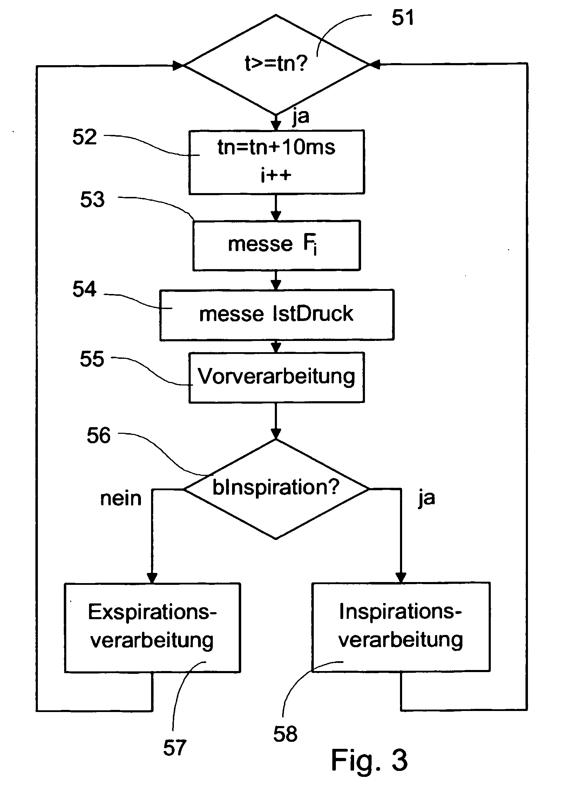

Fig. 3 zeigt ein Flussdiagramm des erfindungsgemäßen Verfahrens. Gemäß einer Ausführungsform wird alle 10 ms in Schritt 53 der Luftfluss $F_i$ und in Schritt 54 der

Istdruck IstDruck gemessen. Der Index i bezeichnet den aktuellen Luftfluss. Da der Solldruck weniger intensiv ausgewertet wird als der Luftfluss, erschien beim Solldruck ein Index entbehrlich. Die aktuelle Zeit wird mit t bezeichnet.

tn bezeichnet die Zeit, zu der die nächste Messung erfolgen soll. In Schritt 51 wird gewartet, bis die nächste Messzeit tn erreicht ist. In Schritt 52 wird tn um 10 ms und i um 1 erhöht.

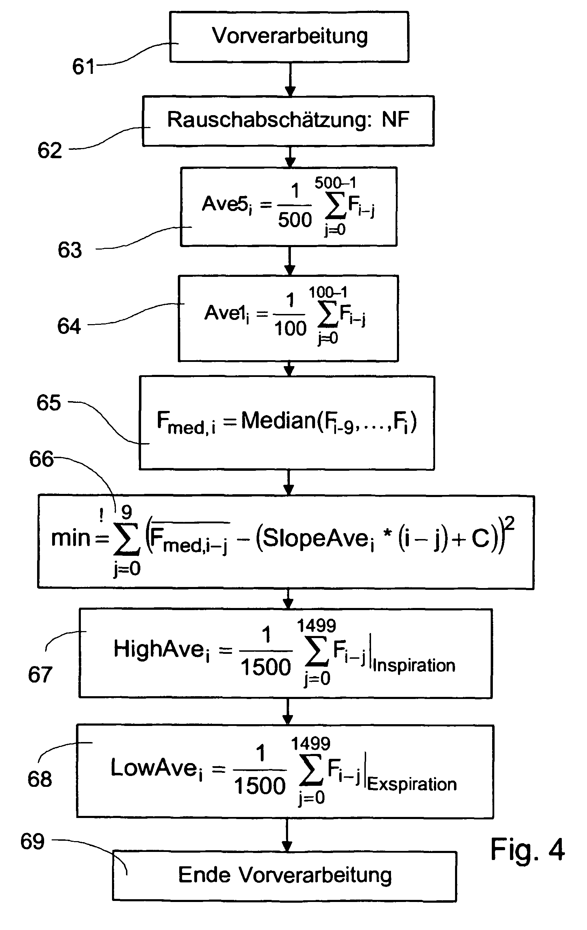

In Schritt 55 erfolgt eine Vorverarbeitung die genauer anhand von Fig. 4 erläutert wird. In Schritt 56 wird geprüft ob sich das Verfahren in einem Inspirations- oder Exspirationsmodus befindet. Dann ist nämlich die boolsche Variable bInspiration wahr bzw. falsch. Im Exspirationsmodus wird die Exspirationsverarbeitung 57 durchgeführt und anschließend in Schritt 51 auf die nächste Messzeit tn gewartet.

Im Inspirationsmodus wird die Inspirationsverarbeitung 58 durchgeführt und anschließend in Schritt 51 gewartet.

Die Vorverarbeitung 55 ist genauer in Fig. 4 dargestellt. Zunächst wird in Schritt 62 eine Rauschabschätzung durchgeführt. Das Ergebnis wird in Variable NF (für "noise flow") gespeichert. In einem einfachen Fall kann die Rauschabschätzung während der Geräteentwicklung erfolgen. In diesem Fall wird in der Variable NF ein konstanter Wert gespeichert. In einer aufwändigeren Ausführungsform kann die Standardabweichung der Luftflussmesswerte $F_i$ aus einem bestimmten Zeitintervall in der Variablen NF gespeichert werden. Die Berechnung der Standardabweichung kann von Zeit zu Zeit wiederholt werden oder gleitend für jedes i erfolgen. Vorteilhafterweise wird ein Zeitintervall ausgewählt, in dem die Ableitung des Luftflusses möglichst nahe bei Null liegt. Dies ist kurz nach dem Einschalten des Geräts oder in der Mitte einer Inspirations- oder Exspirationsphase der Fall.

In Schritt 63 wird ein erster gleitender Mittelwert $Ave5_i$ (Ave=average) über die letzten 500 Luftflussmesswerte $F_i$ gemäß Formel (1) berechnet. Dies entspricht einer Mittelung des Luftflusses über fünf Sekunden.

$$Ave5_i = \frac{1}{500} \sum_{j=0}^{500-1} F_{i-j} \qquad (1)$$

in ähnlicher Weise wird ein weiterer (Ansprüche: dritter) gleitender Mittelwert $Ave1_i$ aus den 100 Luftflussmesswerten der letzten Sekunde gemäß Formel (2) in Schritt 64 berechnet:

$$Ave1_i = \frac{1}{100} \sum_{j=0}^{100-1} F_{i-j} \qquad (2)$$

In Schritt 65 erfolgt eine Medianfilterung über die letzten zehn Luftflussmesswerte gemäß Formel (3). Bei einer Medianfilterung wird der mittlere Wert oder das arithmetische Mittel der beiden mittleren Werte zurückgegeben oder weiterverarbeitet. Eine Medianfilterung ist aufwändiger als eine Mittelwertberechnung. Dafür wird aber das Ergebnis durch Ausreißer praktisch nicht beeinflusst, wohingegen Ausreißer in die Mittelwertberechnung einfließen.

$$F_{med,i} = Median(F_{i-9}, \ldots, F_i) \qquad (3)$$

Anschließend wird ein gleitender Mittelwert $\overline{F_{med,i}}$ über 20 mediangefilterte Werte $F_{med,i}$ in Schritt 66 berechnet. Aus dem gleitenden Mittelwert $\overline{F_{med,i}}$ wird die zeitliche Ableitung des Luftflusses $SlopeAve_i$ geschätzt. Dies kann in einem einfachen Fall dadurch erfolgen, dass $SlopeAve_i$ als Steigung einer Geraden durch zwei gleitende Mittelwerte $\overline{F_{med,i}}$ und $\overline{F_{med,i-k}}$ berechnet wird, die um k*10ms auseinanderliegen. k kann beispielsweise 20 sein. In einer anderen Ausführungsform kann eine Gerade an zehn aufeinander folgende gleitende Mittelwerte $F_{med,i}$ gemäß Formel (4) angepasst werden, wobei die Fehlerquadrate minimiert werden. Die Steigung dieser Geraden $SlopeAve_i$ wird als geschätzte Luftflussableitung interpretiert. In einer weiteren Ausführungsform können auch die absoluten Fehler minimiert werden.

$$min \stackrel{!}{=} \sum_{j=0}^{9} \left( \overline{F_{med,i-j}} - (SlopeAve_i * (i-j) + C) \right)^2 \qquad (4)$$

Schließlich werden in Schritten 67 und 68 noch zwei weitere gleitende Mittelwerte $HighAve_i$ bzw. $LowAve_i$ über je 1500 Luftflussmesswerte $F_i$ gemäß Formel (5) beziehungsweise (6) berechnet, was einer Zeitspanne von 15 Sekunden entspricht. Das Besondere an diesen Mittelungen ist, dass für $HighAve_i$ nur Luftflussmesswerte $F_i$ während der Inspiration und für $LowAve_i$ nur Luftflussmesswerte $F_i$ während der Exspiration berücksichtigt werden. Wenn die Variable bInspiration wahr ist, befindet sich das erfindungsgemäße Verfahren im Inspirationsmodus, sodass die Luftflussmesswerte $F_i$ zur Berechnung von $HighAve_i$ verwendet werden. Andernfalls befindet sich das Verfahren im Exspirationsmodus, sodass die Luftflussmesswerte $F_i$ zur Berechnung von $LowAve_i$ verwendet werden.

$$HighAve_i = \frac{1}{1500} \sum_{j=0}^{1499} F_{i-j}\Big|_{Inspiration} \tag{5}$$

$$LowAve_i = \frac{1}{1500} \sum_{j=0}^{1499} F_{i-j}\Big|_{Exspiration} \tag{6}$$

Anhand von Figuren 5 bis 7 wird die Exspirationsverarbeitung in Schritt 57 erläutert.

In Schritt 72 wird ein so genannter Offset $Off_i$ für jeden Index i gemäß Formel (7) berechnet:

$$Off_i = (HighAve_i - Ave_i(500))/6 \tag{7}$$

Anschließend wird der Speicher $AveHold = Ave1_i$ für drei Sekunden in Schritt 78 gesetzt, wenn $t_{ex} > 1$ s und $F_i > Ave5_i$ und $F_i = Ave1_i$. Letztere drei Bedingungen werden in Schritten 73 bis 75 überprüft. Hieraus ergibt sich, dass auch $Ave1_i > Ave5_i$ ist. $t_{ex}$ bezeichnet hierbei die Zeit seit dem letzten Umschalten in den Exspirationsmodus. Die Zeit, zu der $AveHold = Ave1_i$ gesetzt wird, wird in Speicher $t_{AH0}$ (AH=AveHold) in Schritt 79 gespeichert. Ist eine der in Schritten 73 bis 75 überprüften Bedingungen nicht erfüllt, so wird in Schritt 76 überprüft, ob die drei Sekunden schon abgelaufen sind. Falls ja, wird Speicher $AveHold=0$ in Schritt 77 gesetzt.

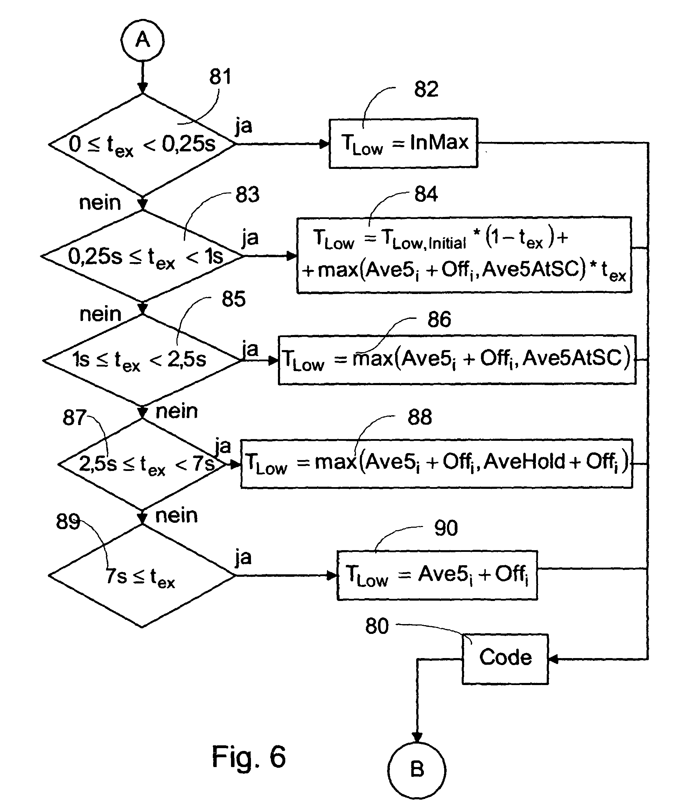

In Fig. 6 wird erläutert wie der Exspirationsschwellenwert $T_{Low}$, der in Figur 1 mit dem Bezugszeichen 3 versehen ist, ermittelt wird. Die Zeit seit dem letzten Umschalten in den Exspirationsmodus $t_{ex}$ wird in Schritten 81, 83, 85, 87 und 89 in fünf Bereiche eingeteilt, die in Figur 1 mit dem Bezugszeichen 14, 15, 16, 17 bzw. 18 versehen sind. Im ersten Bereich, wenn $0 \leq t_{ex} < 0,25$s, wird der Exspirationsschwellenwert $T_{Low}$ gemäß Formel (8) gleich dem Maximum des Luftflusses während der vorausgehenden Inspirationsphase InMax gesetzt, das in Figur 1 mit dem Bezugszeichen 8 versehen ist. Dies verhindert praktisch ein Umschalten in den Inspirationsmodus während der ersten 0,25s.

$$T_{Low} = InMax \quad \text{wenn} \quad 0 \leq t_{ex} < 0,25s \tag{8}$$

Wenn $0,25s \leq t_{ex} < 1s$ wird der Exspirationsschwellenwert $T_{Low}$ während der Zeitspanne 15 näherungsweise zeitlinear gemäß Formel (9) abgesenkt. Ave5AtSC ist gleich dem $Ave5_i$ bei $t_{ex} = 0$, also zum Zeitpunkt des Umschaltens vom Inspirationsmodus in den Exspirationsmodus.

$$T_{Low} = T_{Low,Initial} * (1 - t_{ex}) + \max(Ave5_i + Off_i, Ave5AtSC) * t_{ex} \tag{9}$$

wenn

$$0,25s \leq t_{ex} < 1s$$

Anschließend wird der Exspirationsschwellenwert $T_{Low}$ gemäß der folgenden Formeln 10 bis 12 in Schritten 86, 88 und 90 für die Zeitabschnitte 16, 17 und 18 berechnet.

$$T_{Low} = \max(Ave5_i + Off_i, Ave5AtSC) \text{ wenn } 1s \leq t_{ex} < 2{,}5s \tag{10}$$

$$T_{Low} = \max(Ave5_i + Off_i, AveHold + Off_i) \text{ wenn } 2{,}5s \leq t_{ex} < 7s \tag{11}$$

$$T_{Low} = Ave5_i + Off_i \text{ wenn } 7s \leq t_{ex} \tag{12}$$

Neben dem Exspirationsschwellenwert $T_{Low}$ wird gemäß folgendem C oder JavaScript Code (vgl. JavaScript Das umfassende Referenzwerk, David Flanagan, Übersetzer Ralf Kuhnert et al., O'Reilly, Köln, ISBN 3-930673-56-8) ermittelt, ob der von Teilen der Software vorgegebene Solldruck SollDruck schon erreicht worden ist und der von Drucksensor 36 gemessene Istdruck stabil auf dem Solldruck bleibt. Die Codezeilennummern am rechten Seitenrand gehören nicht zum Code, sondern dienen lediglich der Bezugnahme. Der folgende Code wird in Schritt 80 alle 10 ms durchlaufen.

Ein Umschalten vom Inspirationsmodus in den Exspirationsmodus wird zwar der Solldruck SollDruck stufenförmig abgesenkt, der Istdruck IstDruck bleibt aber zunächst unverändert und sinkt dann allmählich auf den neuen Solldruck. Während dieser Phase ist die Variable DruckErreicht=0. Nach der Prüfung in Codezeile 1, bei der DruckErreicht durch "!" invertiert wird, wird in dieser Phase in Codezeile 2 geprüft ob IstDruck<=SollDruck ist, was noch nicht der Fall ist. Erst bei einem späteren Durchlauf des Codes ist IstDruck auf oder unter SollDruck gefallen. Jetzt wird in Codezeile 2 DruckErreicht inkrementiert, also =1 gesetzt. Beim nächsten Codedurchlauf wird nach der Prüfung in Codezeile 1 DruckErreicht in Codezeile 4 inkrementiert. Dann wird in Codezeile 5 die invertierte Variable DruckStabil geprüft. DruckStabil ist anfangs 0. Deshalb wird anschließend in Codezeile 6 geprüft ob IstDruck>=SollDruck. Wenn der Istdruck IstDruck unter SollDruck überschwingt, ist diese Bedingung bis zum Ende des Überschwingers nicht erfüllt. Beim nächsten Codedurchlauf ist die Bedingung !DruckStabil nicht mehr erfüllt, sodass Codezeile 6 übersprungen wird. DruckErreicht und DruckStabil werden bei den weiteren Codedurchläufen weiter inkrementiert bis die Bedingung IstDruck>SollDruck+UntereDruckSchwelleStabil in Codezeile 7 erfüllt ist. UntereDruckSchwelleStabil ist dabei ein vorgegebener Wert im Bereich von 0,5 bis 1 mbar. Dies kann entweder bei einem zweiten Überschwinger passieren, bei dem der Istdruck wieder um UntereDruckSchwelleStabil über den Solldruck ansteigt. Eine zweite Möglichkeit ist Husten oder Niesen. Die hierbei entstehenden Druckschwankungen sind zu schnell für die Druckregelung des BiLevel-Geräts, sodass sie nicht ausgeregelt werden. Die Variable DruckStabil wird in Schritt 95 ausgewertet um bei Husten oder Niesen ein Umschalten in den Inspirationsmodus zu verhindern.

```
if (!DruckErreicht)                                                         1
        {if (istDruck<=SollDruck) DruckErreicht++; }                        2
else     { 3
        DruckErreicht++;                                                    4
        if (!DruckStabil)                                                   5
                { if (istDruck>=SollDruck) DruckStabil++; }                 6
        else    if (IstDruck>SollDruck+UntereDruckSchwelleStabil)           7
                { DruckErreicht=0; DruckStabil=0; }                         8
        else DruckStabil++;                                                 9
        }                                                                  10
```

Eine der folgenden drei Bedingungen muss erfüllt sein, damit aus dem Exspirationsmodus in den Inspirationsmodus umgeschaltet wird:

1) wenn $F_i > T_{Low}$ && $SlopeAve_i > T_{SlopeUp}$ &&
(! DruckErreicht & &(IstDruck > IstDruck(-9))) oder

2) wenn IstDruck<SollDruck-UntereDruckSchwelleUnstabil && $F_i > F_{i-9} + NF$ oder

3) wenn DruckStabil && IstDruck<SollDruck-UntereDruckSchwelleStabil &&

$$F_i > F_{i-9} + NF.$$

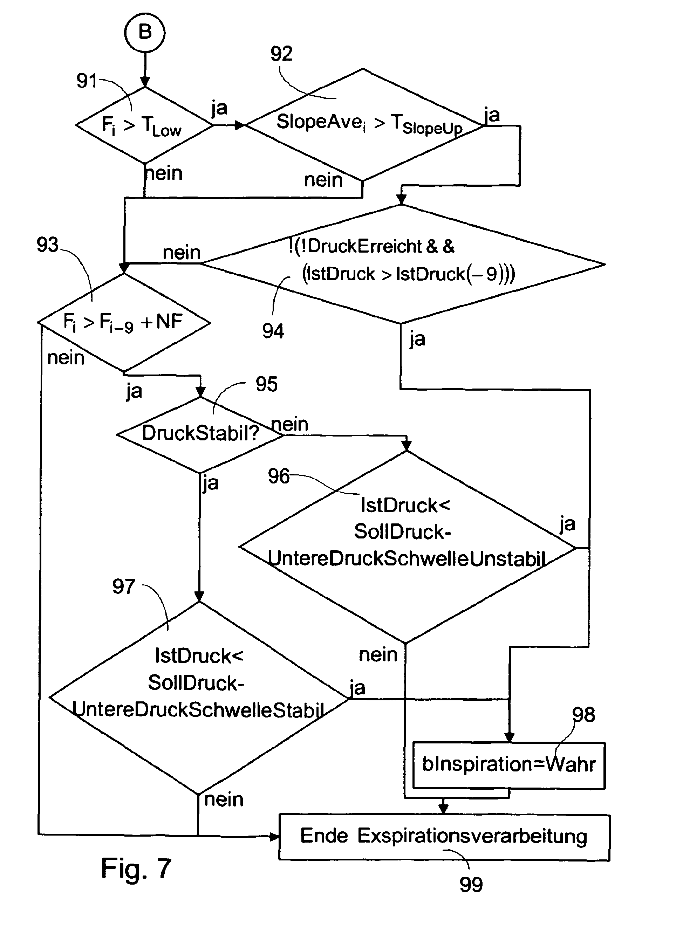

Diese Bedingungen sind auch im Flussdiagramm in Fig. 7 dargestellt. Der übliche Fall für das Umschalten in den Inspirationsmodus ist, dass die Bedingungen in Schritten 91 und 92 erfüllt sind, dass also der Luftfluss $F_i$ größer als der Exspirationsschwellenwert $T_{Low}$ und die Ableitung $SlopeAve_i$ größer als der Schwellenwert $T_{SlopeUp}$ sind. Die in Schritt 94 überprüfte Bedingung (!DruckErreicht & &(IstDruck > IstDruck(-9))) verhindert, dass bei Husten oder Niesen in den Inspirationsmodus umgeschaltet wird. Beim Husten steigt der Istdruck kurzzeitig über den Solldruck an. Hierbei wird in Codezeile 8 DruckErreicht=0 gesetzt. IstDruck(-9) bezeichnet den Istdruck vor 90 ms. -9 ist ein Offset bezogen auf den aktuellen Index. Die 90 ms ergeben sich dann daraus, dass alle 10 ms ein Istdruck gemessen wird. Der Vergleich zwischen dem aktuellen Istdruck und dem Istdruck vor 90 ms zeigt, ob tatsächlich ein Druckanstieg vorliegt.

Die Bedingungen 2 und 3 sind ähnlich strukturiert und werden in Schritten 93 und 95 bis 97 überprüft. Gemäß diesen Bedingungen wird dann in den Inspirationsmodus umgeschaltet, wenn bei starkem Luftholen des Patienten der Luftfluss ansteigt ($F_i > F_{i-9} + NF$) und gleichzeitig der Istdruck unter den Solldruck abfällt. Abhängig von der Variablen DruckStabil wird ein unterschiedlich hoher Abfall des Istdruck unter den Solldruck in Schritten 96 und 97 gefordert. UntereDruckSchwelleUnstabil und UntereDruckSchwelleStabil sind vorbestimmte Konstanten.

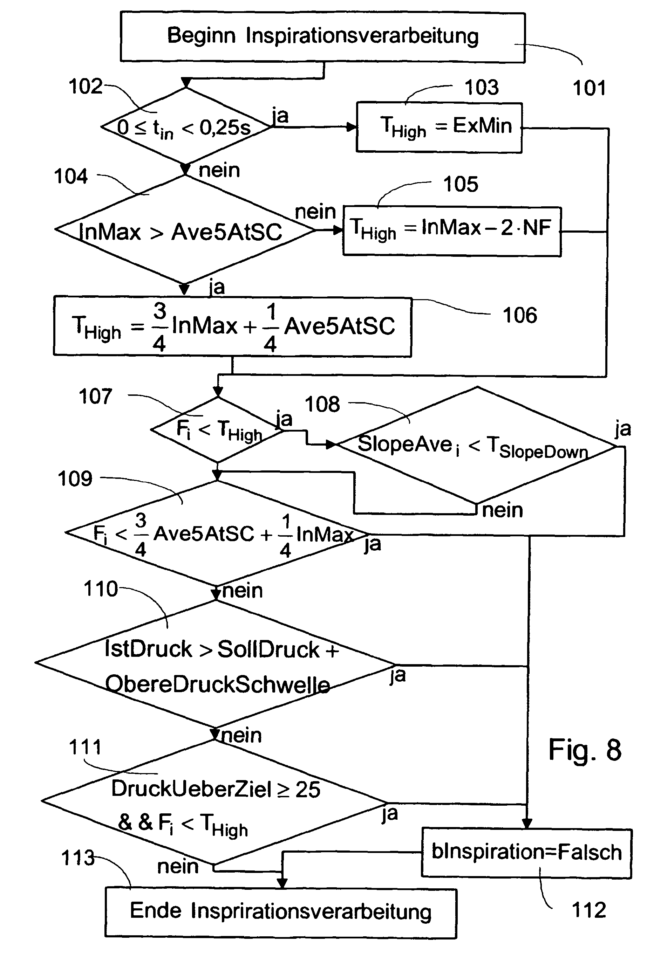

Die in Figur 8 dargestellte Inspirationsverarbeitung ist weniger kompliziert. Es werden nur zwei Zeitbereiche für die Zeit $t_{in}$ seit dem letzten Umschalten in den Inspirationsmodus, nämlich 0 bis 0,25 s und später als 0,25 s in Schritt 102 unterschieden. Innerhalb von 0,25 s nach dem Umschalten in den Inspirationsmodus wird der Inspirationsschwellenwert $T_{High}$, Bezugszeichen 4 gemäß Formel (13) gleich dem Minimum des Luftflusses während der vorausgehenden Exspirationsphase ExMin 7 gesetzt. Dies verhindert praktisch ein Rückspringen in den Exspirationsmodus innerhalb der ersten 0,25 Sekunden.

$$T_{High} = ExMin \text{ wenn } 0 \leq t_{in} < 0{,}25s \qquad (13)$$

Nach Ablauf der ersten 0,25 Sekunden wird in Schritt 104 überprüft ob der bisherige maximale Luftfluss während der aktuellen Inspirationsphase InMax größer als Ave5AtSC ist. Ave5AtSC ist der gleitende Mittelwert über 500 Luftflussmesswerte zum Zeitpunkt des vorausgehenden Umschaltens vom Exspirationsmodus in den Inspirationsmodus. Falls dies der Fall ist, wird der Inspirationsschwellenwert $T_{High}$ gemäß Formel 14 in Schritt 106 berechnet. Andernfalls wird der Inspirationsschwellenwert $T_{High}$ gemäß Formel 15 in Schritt 105 berechnet.

$$T_{High} = \frac{3}{4} InMax + \frac{1}{4} Ave5AtSC \text{ wenn } InMax > Ave5AtSC \qquad (14)$$

$$T_{High} = InMax - 2NF \text{ wenn } InMax \leq Ave5AtSC \qquad (15)$$

Die Bezugszeichen 20, 21, 22 und 23 in Figur 1 beziehen sich auf Zeitabschnitte während einer Inspirationsphase, die durch zwei lokale und ein absolutes Maximum des Luftflusses $F_i$ getrennt sind. Der Inspirationsschwellenwert $T_{High}$ wird mit Überschreiten jedes Maximums weiter nach oben geschoben.

Die Inspirationsphase links in Figur 1 ist weniger interessant. Hier wird der Inspirationsschwellenwert $T_{High}$ vor Erreichen des Maximums 8 des Luftflusses im Zeitabschnitt 12 immer weiter angehoben und bleibt nach Überschreiten des Maximums 8 im Zeitabschnitt 13 konstant.

Anschließend, vor der Entscheidung in Schritt 107 wird der folgende C Code ausgeführt. Wie der Rest der Inspirationsverarbeitung wird auch der Code alle 10 ms durchlaufen. Die Codezeilen 11 bis 20 entsprechen obigen Codezeilen 1 bis 10. Zu beachten ist allerdings, dass der Solldruck SollDruck beim Übergang in die Inspirationsphase stufenförmig angehoben wird, sodass sich am Anfang der Inspirationsphase der Istdruck von unten dem Solldruck annähert. Dies hat zur Folge, dass die Vergleichsoperatoren ">" und "<" gerade vertauscht sind.

In Codezeilen 21 bis 23 wird gemessen, wie lange der Istdruck über dem Solldruck lag. Die Zeit wird in der Variablen DruckUeberZiel herauf gezählt. Der Wert von DruckUeberZiel muss mit 10 ms multipliziert werden, um tatsächlich die Zeit zu erhalten.

```
if (!DruckErreicht)                                                          11
```

```
        { if (IstDruck>=SollDruck) DruckErreicht++; }          12
else    { 13
        DruckErreicht++;                                       14
        if (!DruckStabil)                                      15
               { if (IstDruck<=SollDruck) DruckStabil++; }     16
        else if (IstDruck<SollDruck-ObereDruckSchwelle)        17
               { DruckErreicht=0; DruckStabil=0; }             18
        else DruckStabil++;                                    19
        }                                                      20
if (DruckStabil && (IstDruck>SoIIDruck))                       21
        DruckUeberZiel++;                                      22
else DruckUeberZiel=0;                                         23
```

Eine der folgenden vier Bedingungen muss erfüllt sein, damit aus dem Inspirationsmodus in den Exspirationsmodus umgeschaltet wird:

1) wenn $F_i < T_{High}$ && $SlopeAve_i < T_{SlopeDown}$ oder

2) wenn $F_i < \dfrac{3}{4}\,\text{Ave5AtSC} + \dfrac{1}{4}\,\text{InMax}$ oder

3) wenn IstDruck > SollDruck + ObereDruckSchwelle oder

4) wenn DruckUeberZiel $\geq$ 25 & & $F_i < T_{High}$

Der übliche Fall für das Umschalten in den Exspirationsmodus ist, dass die Bedingungen in Schritten 107 und 108 erfüllt sind, dass also der Luftfluss $F_i$ kleiner als der Inspirationsschwellenwert $T_{High}$ und die Ableitung $SlopeAve_i$ kleiner als der Schwellenwert $T_{SlopeDown}$ sind.

Die zweite Bedingung, die in Schritt 109 überprüft wird, stellt lediglich darauf ab, ob der Luftfluss $F_i$ unter eine vorgegebene Schwelle fällt.

Ferner wird gemäß der dritten Bedingung in den Exspirationsmodus umgeschaltet, wenn der Istdruck um ObereDruckSchwelle über den Solldruck ansteigt. Diese Bedingung wird in Schritt 110 überprüft.

Schließlich wird gemäß der vierten Bedingung in den Exspirationsmodus umgeschaltet, wenn der Istdruck länger als 0,25 s über dem Solldruck liegt und gleichzeitig der Luftfluss $F_i$ unter den Inspirationsschwellenwert $T_{High}$ fällt, was in Schritt 111 überprüft wird.

Beim Umschalten in die Exspiration wird die boolsche Variable bInspiration in Schritt 112 auf falsch gesetzt.

Die Erfindung wurde zuvor anhand von bevorzugten Ausführungsformen näher erläutert. Für einen Fachmann ist jedoch offensichtlich, dass verschiedene Abwandlungen und Modifikationen gemacht werden können, ohne vom Geist der Erfindung abzuweichen. Deshalb wird der Schutzbereich durch die nachfolgenden Ansprüche und ihre Äquivalente festgelegt.

**Patentansprüche**

1.  Verfahren zur Steuerung eines BiLevel-Geräts mit:

   wiederholtem Messen des Luftflusses (1) wobei Luftflussmesswerte erhalten werden;
   Wählen eines Exspirationsschwellenwerts (3);
   Wählen eines Inspirationsschwellenwertes (4);
   wiederholtes Vergleichen des Luftflusses mit dem Exspirationsschwellenwert (3) während eines Exspirationsmoduses (14, 15, 16, 17, 18);
   Umschalten (5) in einen Inspirationsmodus, falls der Luftfluss (1) bei einem Vergleich mit dem Exspirationsschwellenwert (3) größer als der Exspirationsschwellenwert (3) ist;
   wiederholtes Vergleichen des Luftflusses (1) mit dem Inspirationsschwellenwert (4) während des Inspirationsmoduses (11, 12, 13; 19, 20, 21, 22, 23); und
   Umschalten (6) in den Exspirationsmodus, falls der Luftfluss (1) bei einem Vergleich mit dem Inspirationsschwellenwert (4) kleiner als der Inspirationsschwellenwert (4) ist;

**gekennzeichnet durch**:

Wählen des Inspirationsschwellenwerts (4) während einer Zeitspanne (12, 13; 20, 21, 22, 23) kurz nach dem Umschalten in den Inspirationsmodus bis zum Ende des Inspirationsmodus gleich der Differenz aus dem Maximum des Luftflusses während der aktuellen Inspirationsphase minus einem dritten vorbestimmten Wert.

2.  Verfahren gemäß Anspruch 1, **gekennzeichnet durch**:

Speichern des minimalen Luftflusses (7) während des Exspirationsmoduses;
Wählen des Inspirationsschwellenwertes gleich dem gespeicherten minimalen Luftfluss (7) unmittelbar nach dem Umschalten in den Inspirationsmodus;
Speichern des maximalen Luftflusses (8) während des Inspirationsmoduses; und
Wählen des Exspirationsschwellenwertes gleich dem gespeicherten maximalen Luftfluss (8) unmittelbar nach dem Umschalten in den Exspirationsmodus.

3.  Verfahren gemäß einem der obigen Ansprüche, **gekennzeichnet durch**:

Berechnen einer Luftflussableitung (2) **durch** Schätzen der zeitlichen Ableitung des Luftflusses (1);
Vergleichen der Luftflussableitung (2) mit einem Exspirationsableitungsschwellenwert während eines Exspirationsmoduses;
Umschalten (5) in den Inspirationsmodus nur dann, wenn zusätzlich die Luftflussableitung (2) größer als der Exspirationsableitungsschwellenwert ist;
Vergleichen der Luftflussableitung mit einem Inspirationsableitungsschwellenwert während eines Inspirationsmoduses;
Umschalten (6) in den Exspirationsmodus nur dann, wenn zusätzlich die Luftflussableitung größer als der Inspirationsableitungsschwellenwert ist.

4.  Verfahren gemäß Anspruch 3, **dadurch gekennzeichnet, dass** das Berechnen der Luftflussableitung enthält:

wiederholtes Berechnen des Medians aus einer ersten vorbestimmten Anzahl von aufeinanderfolgenden Luftflussmesswerten, wobei Medianwerte erhalten werden;
Berechnen eines gemittelten Luftflusses als arithmetisches Mittel einer zweiten vorgegebenen Anzahl von Medianwerten; und
Berechnen der zeitlichen Ableitung des gemittelten Luftflusses um die Luftflussableitung (2) zu erhalten.

5.  Verfahren gemäß Anspruch 3 oder 4, **dadurch gekennzeichnet, dass** die Luftflussableitung (2) vor dem Vergleichen mit dem Exspirations- und Inspirationsableitungsschwellenwert über eine erste vorbestimmte Zeitspanne gemittelt wird.

6.  Verfahren gemäß einem der obigen Ansprüche, **dadurch gekennzeichnet, dass** nicht vom Exspirationsmodus in den Inspirationsmodus umgeschaltet wird, wenn der Istdruck noch nicht auf den Solldruck eingeschwungen ist und der aktuelle Istdruck über dem Istdruck liegt, der vor einer zweiten vorgegebenen Zeitspanne vor der aktuellen Zeit gemessen wurde.

7.  Verfahren gemäß einem der obigen Ansprüche, **dadurch gekennzeichnet, dass** vom Exspirationsmodus in den Inspirationsmodus umgeschaltet wird, wenn der Istdruck (36) um einen ersten vorgegebenen Wert unter dem Solldruck liegt und der aktuelle Luftfluss (1) um einen zweiten vorgegebenen Wert über dem Luftfluss liegt, der vor einer dritten vorgegebenen Zeitspanne vor der aktuellen Zeit liegt.

8.  Verfahren gemäß einem der obigen Ansprüche, **gekennzeichnet durch** die Schritte:

Berechnen eines ersten gleitenden Mittelwerts über eine dritte vorbestimmte Anzahl von Luftflussmesswerten;
Berechnen eines zweiten gleitenden Mittelwerts über eine vierte vorbestimmte Anzahl von Luftflussmesswerten, die ausschließlich während einem Inspirationsmodus gemessen wurden.

9.  Verfahren gemäß Anspruch 8, **gekennzeichnet durch** die Schritte:

Absenken des Exspirationsschwellenwertes während einer sechsten Zeitspanne (16) kurz nach dem Umschalten in den Exspirationsmodus von einem maximalen Luftfluss (8), der während der vorangehenden Inspirationsphase gemessen wurde, auf die Summe aus dem mit einem ersten Faktor gewichteten ersten gleitenden Mittelwert plus den mit einem zweiten Faktor gewichteten zweiten gleitenden Mittelwert, wobei der ersten Faktor etwas kleiner als eins und die Summe aus ersten und zweiten Faktor gleich eins ist; und
Wählen des Exspirationsschwellenwertes gleich der Summe im Anschluss an die sechste Zeitspanne.

10. Verfahren gemäß Anspruch 9, **dadurch gekennzeichnet, dass** der Exspirationsschwellenwert während der sechsten Zeitspanne nur auf einen Umschaltmittelwert abgesenkt wird, wenn der Umschaltmittelwert größer als die Summe ist, und der Exspirationsschwellenwert während einer siebten Zeitspanne (16, 17, 18) im Anschluss an die sechste Zeitspanne (15) gleich dem Umschaltmittelwert gewählt wird, wenn der Umschaltmittelwert größer als die Summe ist, wobei der Umschaltmittelwert gleich dem ersten gleitenden Mittelwert zum Zeitpunkt des vorangehenden Umschaltens in den Exspirationsmodus ist.

11. Verfahren gemäß einem der Ansprüche 8 bis 10, **gekennzeichnet durch** die Schritte:

Berechnen eines dritten gleitenden Mittelwerts über eine fünfte vorbestimmte Anzahl von Luftmesswerten;
Speichern des dritten gleitenden Mittelwerts während der siebten Zeitspanne für eine achte Zeitspanne, die kürzer als die siebte Zeitspanne ist, in einem Speicher, wenn sowohl der Luftfluss als auch der dritte gleitende Mittelwert größer als der erste gleitende Mittelwert sind und der Luftfluss gleich dem dritten gleitenden Mittelwert ist; andernfalls speichern von Null in dem Speicher;
Berechnen der Differenz vom zweiten gleitenden Mittelwert minus dem ersten gleitenden Mittelwert;
Multiplizieren der Differenz mit einem dritten Faktor um ein Produkt zu erhalten; und
Addieren des Produkts mit dem Wert im Speicher um den Exspirationsschwellenwert zu erhalten.

12. Verfahren gemäß Anspruch 10 oder Anspruch 11, so weit sich Anspruch 11 auf Anspruch 10 rückbezieht, **dadurch gekennzeichnet, dass** der Inspirationsschwellenwert (4) während einer Zeitspanne (12, 13; 20, 21, 22, 23) kurz nach dem Umschalten in den Inspirationsmodus bis zum Ende des Inspirationsmodus gleich dem mit einem vierten Faktor multiplizierten Maximum des Luftflusses während der aktuellen Inspirationsphase minus dem mit einem fünften Faktor multiplizierten Umschaltmittelwert gewählt wird, wobei der vierte Faktor etwas kleiner als eins und die Summe aus dem viertem Faktor plus dem fünften Faktor gleich eins ist.

13. Verfahren gemäß Anspruch 12 , **dadurch gekennzeichnet, dass** der Inspirationsschwellenwert während der Zeitspanne (12, 13; 20, 21, 22, 23) gemäß Anspruch 12 gewählt wird, wenn das Maximum des Luftflusses während der aktuellen Inspirationsphase größer als der Umschaltmittelwert ist und andernfalls der Inspirationsschwellenwert während dieser Zeitspanne (12, 13; 20, 21, 22, 23) gemäß Anspruch 1 gewählt wird.

14. Beatmungsgerät mit:

einem Lüfter (40);
einem Drucksensor (36) zur Bestimmung des Überdrucks, unter dem Luft vom Lüfter (40) geliefert wird;
einem Flusssensor (38) zur Messung des Luftflusses (1);
einem Prozessor (39), der mit dem Drucksensor (36) und dem Flusssensor (38) verbunden ist und dem ein Drucksignal vom Drucksensor (36) und ein Luftflusssignal vom Flusssensor (38) zugeführt wird, wobei der Prozessor (39) eine Folge von Befehlen speichert, sodass der Prozessor (39) im Betrieb ein Verfahren gemäß einem der obigen Ansprüche durchführt.

**Claims**

1. Method for controlling a bi-level apparatus, comprising:

repeatedly measuring the airflow (1), thereby obtaining measured airflow values;
choosing an expiration threshold value (3);
choosing an inspiration threshold value (4);
repeatedly comparing the airflow with the expiration threshold value (3) during an expiration mode (14, 15, 16, 17, 18);
switching (5) to an inspiration mode if the airflow (1) is greater than the expiration threshold value (3) in a comparison with the expiration threshold value (3);

repeatedly comparing the airflow (1) with the inspiration threshold value (4) during the inspiration mode (11, 12, 13; 19, 20, 21, 22, 23); and

switching (6) to the expiration mode if the airflow (1) is smaller than the inspiration threshold value (4) in a comparison with the inspiration threshold value (4);

**characterized by**:

choosing the inspiration threshold value (4) during a time span (12, 13; 20, 21, 22, 23) shortly after the switching to the inspiration mode up to the end of the inspiration mode to be equal to the difference from the maximum of the airflow during the current inspiration phase minus a third predetermined value.

2. Method according to claim 1, **characterized by**:

storing the minimum airflow (7) during the expiration mode;
choosing the inspiration threshold value equal to the stored minimum airflow (7) immediately after switching to the inspiration mode;
storing the maximum airflow (8) during the inspiration mode; and
choosing the expiration threshold value equal to the stored maximum airflow (8) immediately after switching to the expiration mode.

3. Method according to one of the preceding claims, **characterized by**:

calculating an airflow derivative (2) by estimating the time derivative of the airflow (1);
comparing the airflow derivative (2) with an expiration derivative threshold value during an expiration mode;
switching (5) to the inspiration mode only if additionally the airflow derivative (2) is greater than the expiration derivative threshold value;
comparing the airflow derivative with an inspiration derivative threshold value during an inspiration mode;
switching (6) to the expiration mode only if additionally the airflow derivative is greater than the inspiration derivative threshold value.

4. Method according to claim 3, **characterized in that** calculating the airflow derivative includes:

repeatedly calculating the median from a first predetermined number of successive measured airflow values, thereby obtaining median values;
calculating an averaged airflow as arithmetic average of a second given number of median values; and
calculating the time derivative of the averaged airflow to obtain the airflow derivative (2).

5. Method according to claim 3 or 4, **characterized in that** the airflow derivative (2) is averaged over a first predetermined time span prior to the comparison with the expiration and inspiration derivative threshold value.

6. Method according to one of the preceding claims, **characterized in that** no switching from the expiration mode to the inspiration mode takes place if the actual pressure is not yet settled on the set pressure and the current actual pressure is above the actual pressure measured before a second given time span prior to the current time.

7. Method according to one of the preceding claims, **characterized in that** switching from the expiration mode to the inspiration mode takes place if the actual pressure (36) is below the set pressure by a first given value and the current airflow (1) is above the airflow preceding a third given time span prior to the current time by a second given value.

8. Method according to one of the preceding claims, **characterized by** the steps:

calculating a first moving average value by a third predetermined number of measured airflow values;
calculating a second moving average value by a fourth predetermined number of measured airflow values measured exclusively during an inspiration mode.

9. Method according to claim 8, **characterized by** the steps:

lowering the expiration threshold value during a sixth time span (16) shortly after switching to the expiration mode from a maximum airflow (8) measured during the preceding inspiration phase to the sum of the first moving average value weighted by a first factor plus the second moving average value weighted by a second factor, wherein the first factor is slightly smaller than one and the sum of the first and second factor is equal to one; and

choosing the expiration threshold value equal to the sum following the sixth time span.

10. Method according to claim 9, **characterized in that** the expiration threshold value is only lowered to a switching average value during the sixth time span if the switching average value is greater than the sum, and the expiration threshold value is chosen to be equal to the switching average value during a seventh time span (16, 17, 18) following the sixth time span (15) if the switching average value is greater than the sum, wherein the switching average value is equal to the first moving average value at the moment of the preceding switching to the expiration mode.

11. Method according to one of claims 8 to 10, **characterized by** the steps:

calculating a third moving average value over a fifth predetermined number of measured airflow values; storing the third moving average value during the seventh time span for an eighth time span being shorter than the seventh time span in a memory if both the airflow and the third moving average value are greater than the first moving average value and the airflow is equal to the third moving average value; otherwise storing of zero in the memory; calculating the difference of the second moving average value minus the first moving average value; multiplying the difference by a third factor to obtain a product; and adding the product to the value in the memory to obtain the expiration threshold value.

12. Method according to claim 10 or claim 11, in as far as claim 11 depends on claim 10, **characterized in that** during a time span (12, 13; 20, 21, 22, 23) shortly after the switching to the inspiration mode up to the end of the inspiration mode the inspiration threshold value (4) is chosen to be equal to the maximum of the airflow during the current inspiration phase multiplied by a fourth factor minus the switching average value multiplied by a fifth factor, wherein the fourth factor is slightly smaller than one and the sum of the fourth factor plus the fifth factor is equal to one.

13. Method according to claim 12, **characterized in that** during the time span (12, 13; 20, 21, 22, 23) the inspiration threshold value is chosen according to claim 12 if the maximum of the airflow during the current inspiration phase is greater than the switching average value, while otherwise the inspiration threshold value during this time span (12, 13; 20, 21, 22, 23) is chosen according to claim 1.

14. Respiratory apparatus, comprising:

a fan (40); a pressure sensor (36) for determining the overpressure under which air is provided by the fan (40); a flow sensor (38) for measuring the airflow (1); a processor (39) connected to the pressure sensor (36) and the flow sensor (38), to which a pressure signal is fed from the pressure sensor (36) and an airflow signal from the flow sensor (38), wherein the processor (39) stores a sequence of instructions so that the processor (39) performs a method according to one of the preceding claims during operation.

**Revendications**

1. Procédé de commande d'un appareil BiLevel comprenant :

la mesure répétée du flux d'air (1), les valeurs mesurées du flux d'air étant obtenues par détermination ; le choix d'une valeur seuil d'expiration (3) ; le choix d'une valeur seuil d'inspiration (4) ; la comparaison répétée du flux d'air avec la valeur seuil d'expiration (3) pendant un mode d'expiration (14, 15, 16, 17, 18) ; la commutation (5) dans un mode d'inspiration si le flux d'air (1) est supérieur à la valeur seuil d'expiration (3) lors d'une comparaison avec la valeur seuil d'expiration (3) ; la comparaison répétée du flux d'air (1) avec la valeur seuil d'inspiration (4) pendant le mode d'inspiration (11, 12, 13 ; 19, 20, 21, 22, 23) ; et

la commutation (6) en mode d'expiration si le flux d'air (1) est inférieur à la valeur seuil d'inspiration (4) lors d'une comparaison avec la valeur seuil d'inspiration (4) ;
**caractérisé par** :

le choix d'une valeur seuil d'inspiration (4) pendant un intervalle de temps (12, 13; 20, 21, 22, 23) suivant de peu la commutation en mode d'inspiration jusqu'à la fin du mode d'inspiration égale à la différence du maximum du flux d'air pendant la phase d'inspiration actuelle moins une troisième valeur prédéfinie.

**2.** Procédé selon la revendication 1, **caractérisé par** :

la mémorisation du flux d'air (7) minimal en mode d'expiration ;
le choix de la valeur seuil d'inspiration égale au flux d'air (7) minimal mémorisé directement après la commutation en mode d'inspiration ;
la mémorisation du flux d'air (8) maximal en mode d'inspiration ; et
le choix de la valeur seuil d'expiration égale au flux d'air (8) maximal mémorisé directement après la commutation en mode d'expiration.

**3.** Procédé selon l'une quelconque des revendications précédentes, **caractérisé par** :

le calcul d'une dérivée du flux d'air (2) en estimant la dérivée dans le temps du flux d'air (1) ;
la comparaison de la dérivée du flux d'air (2) avec une valeur seuil de dérivée d'expiration dans un mode d'expiration ;
la commutation (5) en mode d'inspiration seulement lorsque la dérivée de flux d'air (2) est en outre supérieure à la valeur seuil de dérivée d'expiration ;
la comparaison de la dérivée de flux d'air avec une valeur seuil de dérivée d'inspiration dans un mode d'inspiration ;
la commutation (6) en mode d'expiration seulement lorsque la dérivée de flux d'air est en outre supérieure à la valeur seuil de dérivée d'inspiration.

**4.** Procédé selon la revendication 3, **caractérisé en ce que** le calcul de la dérivée de flux d'air contient :

le calcul répété de la médiane à partir d'un premier nombre prédéfini de valeurs mesurées du flux d'air se suivant, les valeurs médianes étant obtenues par détermination ;
le calcul d'un flux d'air moyen comme la moyenne arithmétique d'un deuxième nombre prédéfini de valeurs médianes ; et
le calcul de la dérivée dans le temps du flux d'air moyen pour obtenir par détermination la dérivée du flux d'air (2).

**5.** Procédé selon la revendication 3 ou 4, **caractérisé en ce que** la dérivée de flux d'air (2) est moyennée avant la comparaison avec la valeur seuil de dérivée d'inspiration et d'expiration sur un premier intervalle de temps prédéfini.

**6.** Procédé selon l'une quelconque des revendications précédentes, **caractérisé en ce que** l'on ne commute pas du mode d'expiration en mode d'inspiration tant que la pression réelle n'atteint pas la pression théorique et que la pression réelle actuelle est supérieure à la pression réelle mesurée avant un deuxième intervalle de temps prédéfini précédant l'instant actuel.

**7.** Procédé selon l'une quelconque des revendications précédentes, **caractérisé en ce que** l'on commute du mode d'expiration en mode d'inspiration lorsque la pression réelle (36) est inférieure à la pression théorique d'une première valeur prédéfinie et que le flux d'air (1) actuel se situe au-dessus du flux d'air d'une deuxième valeur prédéfinie, avant un troisième intervalle de temps prédéfini précédant l'instant actuel.

**8.** Procédé selon l'une quelconque des revendications précédentes, **caractérisé par** les étapes de :

calcul d'une première valeur moyenne glissante sur un troisième nombre prédéfini de valeurs mesurées du flux d'air ;
calcul d'une deuxième valeur moyenne glissante sur un quatrième nombre prédéfini de valeurs mesurées du flux d'air, mesurées exclusivement pendant un mode d'inspiration.

**9.** Procédé selon la revendication 8, **caractérisé par** les étapes de :

baisse de la valeur seuil d'expiration pendant un sixième intervalle de temps (16) peu après la commutation en mode d'expiration d'un flux d'air (8) maximal mesuré pendant la phase d'inspiration précédente, jusqu'à atteindre la somme de la première valeur moyenne glissante pondérée par un premier facteur plus la seconde valeur moyenne glissante pondérée par un second facteur, le premier facteur étant quelque peu inférieur à un et la somme du premier et du second facteur étant égale à un ; et

choix de la valeur seuil d'expiration égale à la somme obtenue à la suite du sixième intervalle de temps.

10. Procédé selon la revendication 9, **caractérisé en ce que** la valeur seuil d'expiration pendant le sixième intervalle de temps ne baisse que jusqu'à une valeur moyenne de commutation lorsque la valeur moyenne de commutation est supérieure à la somme et que la valeur seuil d'expiration pendant un septième intervalle de temps (16, 17, 18) suivant le sixième intervalle de temps (15) est choisie égale à la valeur moyenne de commutation, lorsque la valeur moyenne de commutation est supérieure à la somme, la valeur moyenne de commutation étant égale à la première valeur moyenne glissante au moment de la commutation précédente en mode d'expiration.

11. Procédé selon l'une quelconque des revendications 8 à 10, **caractérisé par** les étapes de :

calcul d'une troisième valeur moyenne glissante sur un cinquième nombre prédéfini de valeurs mesurées de flux d'air ;

mémorisation de la troisième valeur moyenne glissante pendant le septième intervalle de temps pour un huitième intervalle de temps plus court que le septième intervalle de temps, dans une mémoire, quand aussi bien le flux d'air que la troisième valeur moyenne glissante sont supérieurs à la première valeur moyenne glissante et que le flux d'air est égal à la troisième valeur moyenne glissante ; sinon mémorisation de zéro dans la mémoire ;

calcul de la différence de la troisième valeur moyenne glissante moins la première valeur moyenne glissante ;

multiplication de la différence avec un troisième facteur pour obtenir un produit ; et

addition du produit à une valeur dans la mémoire pour obtenir la valeur seuil d'expiration.

12. Procédé selon la revendication 10 ou la revendication 11, dans la mesure où la revendication 11 se rapporte à la revendication 10, **caractérisé en ce que** la valeur seuil d'inspiration (4) pendant un intervalle de temps (12, 13 ; 20, 21, 22, 23) suivant de peu la commutation en mode d'inspiration jusqu'à la fin du mode d'inspiration est choisie égale au maximum du flux d'air multiplié par un quatrième facteur pendant la phase d'inspiration actuelle moins la valeur moyenne de commutation multipliée par un cinquième facteur, le quatrième facteur étant quelque peu inférieur à un et la somme du quatrième facteur et du cinquième facteur étant égale à un.

13. Procédé selon la revendication 12, **caractérisé en ce que** la valeur seuil d'inspiration pendant l'intervalle de temps (12, 13 ; 20, 21, 22, 23) selon la revendication 12 est choisie lorsque le maximum du flux d'air pendant la phase d'inspiration actuelle est supérieur à la valeur moyenne de commutation et sinon, la valeur seuil d'inspiration pendant cet intervalle de temps (12, 13 ; 20, 21, 22, 23) est choisie selon la revendication 1.

14. Appareil de respiration avec :

une soufflante (40) ;

un capteur de pression (36) pour déterminer la surpression à laquelle l'air est sorti de la soufflante (40) ;

un capteur de flux (38) pour mesurer le flux d'air (1) ;

un processeur (39) qui est relié au capteur de pression (36) et au capteur de flux (38) et auquel est amené un signal de pression provenant du capteur de pression (36) et un signal de flux d'air provenant du capteur de flux d'air (38), le processeur (39) mémorisant une suite de commandes, de façon à ce que le processeur (39) mette en oeuvre en fonctionnement un procédé selon l'une quelconque des revendications précédentes.

EP 1 727 580 B1

$t_{0,25s<durationExsp<1s}$

$t_{2,5s<durationExsp<7s}$

$t_{7s<durationExsp}$

$t_{1s<durationExsp<2,5s}$

$t_{0s<durationExsp<0,25s}$

$t_{0s<durationInsp<0,25s}$

$t_{0,25s<durationInsp}$

Fig.1

Fig.2

51

t>=tn?

ja

52

tn=tn+10ms
i++

53

messe $F_i$

54

messe IstDruck

55

Vorverarbeitung

56

bInspiration?

nein                ja

Exspirationsverarbeitung

Inspirationsverarbeitung

57                58

Fig. 3

Vorverarbeitung

Rauschabschätzung: NF

$$Ave5_i = \frac{1}{500} \sum_{j=0}^{500-1} F_{i-j}$$

$$Ave1_i = \frac{1}{100} \sum_{j=0}^{100-1} F_{i-j}$$

$$F_{med,i} = Median(F_{i-9}, \ldots, F_i)$$

$$min \overset{!}{=} \sum_{j=0}^{9} \left( \overline{F_{med,i-j}} - (SlopeAve_i * (i-j) + C) \right)^2$$

$$HighAve_i = \frac{1}{1500} \sum_{j=0}^{1499} F_{i-j} \big|_{Inspiration}$$

$$LowAve_i = \frac{1}{1500} \sum_{j=0}^{1499} F_{i-j} \big|_{Exspiration}$$

Ende Vorverarbeitung

Fig. 4

Beginn Exspirationsverarbeitung —— 71

$Off_i = (HighAve_i - Ave5_i)/6$ —— 72

73 — $t_{ex} > 1s$ —ja→ 74 $F_i > Ave5_i$ —ja→ 75 $F_i = Ave1_i$ —ja→

nein                nein              nein

$t - t_{AH0} > 3s$ —— 76    nein

78 — AveHold = $Ave_i(100)$

79 —— $t_{AH0} = t$

ja

AveHold=0 —— 77

(A)

Fig. 5

Ⓐ

81

$0 \leq t_{ex} < 0,25s$ → ja → 82 $T_{Low} = InMax$

nein ↓

83

$0,25s \leq t_{ex} < 1s$ → ja → 84 $T_{Low} = T_{Low,Initial} * (1 - t_{ex}) + + \max(Ave5_i + Off_i, Ave5AtSC) * t_{ex}$

nein ↓

85

$1s \leq t_{ex} < 2,5s$ → ja → 86 $T_{Low} = \max(Ave5_i + Off_i, Ave5AtSC)$

87

nein ↓

$2,5s \leq t_{ex} < 7s$ → ja → 88 $T_{Low} = \max(Ave5_i + Off_i, AveHold + Off_i)$

89

nein ↓

$7s \leq t_{ex}$ → ja → 90 $T_{Low} = Ave5_i + Off_i$

80

Code

Fig. 6

Ⓑ

B

91

$F_i > T_{Low}$

ja

92

$SlopeAve_i > T_{SlopeUp}$

ja

nein

nein

93

nein

$!(!DruckErreicht \&\&$
$(IstDruck > IstDruck(-9)))$

94

$F_i > F_{i-9} + NF$

nein

ja

ja

95

DruckStabil?

nein

ja

96

IstDruck<
SollDruck-
UntereDruckSchwelleUnstabil

ja

97

IstDruck<
SollDruck-
UntereDruckSchwelleStabil

ja

nein

nein

98

bInspiration=Wahr

nein

Ende Exspirationsverarbeitung

Fig. 7

99

EP 1 727 580 B1

Fig. 8

Beginn Inspirationsverarbeitung — 101

102: $0 \leq t_{in} < 0{,}25s$ — ja → $T_{High} = ExMin$ (103)

nein

104: $InMax > Ave5AtSC$ — nein → $T_{High} = InMax - 2 \cdot NF$ (105)

ja

106: $T_{High} = \dfrac{3}{4} InMax + \dfrac{1}{4} Ave5AtSC$

107: $F_i < T_{High}$ — ja → 108: $SlopeAve_i < T_{SlopeDown}$ — ja

nein

109: $F_i < \dfrac{3}{4} Ave5AtSC + \dfrac{1}{4} InMax$ — ja

nein

110: $IstDruck > SollDruck + ObereDruckSchwelle$ — ja

nein

111: $DruckUeberZiel \geq 25$ && $F_i < T_{High}$ — ja

nein

112: bInspiration=Falsch

113: Ende Inspirationsverarbeitung

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19849571 A1
- WO 0024446 A1
- WO 0200283 A1
- WO 02083221 A1
- WO 9835715 A1
- EP 0656216 A2
- WO 02083221 A2
- US 5433193 A

**In der Beschreibung aufgeführte Nicht-Patentliteratur**

- *Chest,* Oktober 1996, vol. 110, 1077-1088
- *Sleep,* vol. 19, 184-188
- **DAVID FLANAGAN ; ÜBERSETZER RALF KUH-NERT et al.** JavaScript Das umfassende Referenzwerk